# EUROPEAN PATENT SPECIFICATION

(11) **EP 0 385 001 B1**
(45) Date of publication and mention of the grant of the patent: **26.04.1995**
(21) Application number: 89301986.9
(22) Date of filing: 28.02.1989
(51) Int. Cl.: A61B 17/22

(54) **An apparatus and method for disintegrating calculus**
Vorrichtung und Verfahren zur Zerkleinerung von Konkrementen
Dispositif et procédé de fragmentation de calculs

(43) Date of publication of application: 05.09.1990
(73) Proprietor: KABUSHIKI KAISHA TOSHIBA, Kawasaki-shi, Kanagawa-ken 210 (JP)
(72) Inventor: Aida, Satoshi, Minato-ku Tokyo (JP); Iwama, Nobuyuki, Minato-ku Tokyo (JP)
(74) Representative: BATCHELLOR, KIRK & CO.

(56) References cited:
- EP-A- 0 148 653
- WO-A-87/01927
- FR-A- 2 591 467
- GB-A- 2 187 840
- US-A- 4 610 249

## Description

This invention relates to an apparatus and a method of using the apparatus for the non-invasive treatment of a concretion or calculus in the human body or using shockwave energy and more particularly to an apparatus and method which more accurately and reliably confirms the location of the calculus using sound.

Recently, an apparatus for disintegrating calculus or kidney stones (sometimes known as a lithotrity) using high powered pulsed ultrasonic waves (hereinafter called ultrasonic shockwave) has attracted attention, because it has advantages in size and cost compared with a lithotrity using other shockwave energy which is used in practice. A lithotrity using other shockwave energy is shown for example in US-A- 4,610,249, and a lithotrity using ultrasonic shockwave is shown for example in US-A-4,617,931.

A lithotrity using ultrasonic shockwaves, (hereinafter called an ultrasonic lithotrity), generally has a main ultrasonic transducer which has a spherical surface forming a focal point in its geometric centre to disintegrate calculus, and an imaging ultrasonic transducer or other imaging device for obtaining a cross sectional image of a patient, disposed a certain distance from the main ultrasonic transducer. After the focal point of the main ultrasonic transducer is localised to the calculus using the image obtained by the imaging transducer, a high power ultrasonic shockwave is projected at the calculus by the main ultrasonic transducer so as to destroy it. However, precise localisation of the focal point of the main ultrasonic transducer to the calculus is very difficult especially using only the cross sectional image of the calculus obtained by the image transducer.This is because the position of the calculus in the cross sectional image and the focal point of the main transducer do not always coincide. Further, the emission of the high power ultrasonic shockwave is usually repeated to completely disintegrate the calculus, and the patient moves during the emission period due, for example, to breathing. Thus, the high power shockwave is not always focused on the calculus, but can focus on neighbouring normal human organs or tissue, and this causes harmful effects there.

While the above conventional apparatus depends on the eyesight of the operator to localise the focal point of the main transducer on the calculus, there is an apparatus which depends on the auditory sense of the operator, an example being shown in Japanese Patent Application (KOKAI) 62-49843. This apparatus emits a weak ultrasonic shockwave from the main ultrasonic transducer before disintegrating the calculus, i.e., before emitting a high power ultrasonic shockwave. The weak ultrasonic shockwave is reflected from the calculus and its echo signal is received by the main ultrasonic transducer. A detector is provided which selectively detects such echo signals that are only reflected from near the focal point of the main ultrasonic transducer. The selected echo signals are then transformed into a sound signal and supplied to a loud speaker, so that the operator may adjust the focal point of the main ultrasonic transducer to the calculus, by searching for the position where the sound becomes a maximum.

However, according to the above apparatus, the main ultrasonic transducer must emit both high power and weak ultrasonic shockwave, and also receive the echo signals corresponding to the weak ultrasonic shockwave. Thus, the apparatus becomes complicated and large in size. And the apparatus still depends upon the operator to precisely set the focal point of the main transducer to the calculus.

It is therefore an object of this invention to provide an apparatus and method for disintegrating calculus which is easy for the operator to operate , small in size and safe for patients.

It is another object of this invention to provide ultrasonic lithotrity apparatus and a method for operating the apparatus which can effectively and precisely confirm that the calculus is exactly at the focal point of the main ultrasonic transducer.

It is another object of this invention to provide ultrasonic lithotrity apparatus and method which can prohibit the emission of the ultrasonic shockwaves for disintegrating calculus when its position is not coincident with the focal point of the transducer.

These objects are accomplished by this invention, which is based upon the experience that when a high power ultrasonic wave hits a calculus, audio sound is generated by the calculus with an inherent audio frequency.

The reader may be further enlightened as to the significance of the present invention by reference to the prior art disclosure in GB-A-2 187 840, with respect to which claim 1 of this application is characterised.

Accordingly the present invention provides an apparatus for disintegrating calculus in a living body using ultrasonic shockwaves comprising:
ultrasonic transducer means for emitting first ultrasonic shockwaves having a first energy level for location of the calculus, and for emitting second ultrasonic shockwaves having an energy level, greater than the first energy level, to disintegrate it;
sound receiver means for receiving sound waves from the living body and transforming the sound waves into electrical signals, said sound waves being generated by the calculus in response to said first ultrasonic shockwave; and
confirmation means, coupled to said sound receiver means for confirming the position of the calculus at the focal point of the ultrasonic transducer means by using said electrical signal derived from said sound receiver means;
control means, coupled between said confirmation means and said ultrasonic transducer means for prohibiting the emission of the second ultrasonic shockwave when said confirmation means has determined that the position of the calculus is not at the focal point of the ultrasonic transducer means characterised in that said confirmation means includes frequency analysis means for analysing the frequency components corresponding to the inherent frequency of the audio sounds generated by the calculus responding to the emission of the first ultrasonic shockwaves and comparing means for comparing said frequency components with a predetermined threshold value.

According to a second aspect of the present invention there is provided a method of operating an apparatus for disintegrating calculus comprising the steps of:
projecting a harmlessly low powered focused shockwave from an ultrasonic transducer means,
receiving sound waves with a receiver directed towards the target on which the ultrasonic transducer means is focused and converting the received sound waves into electrical signals,
transmitting the signals to a confirming means,
prohibiting the emission of a second, high energy, calculus disintegrating shockwave from the ultrasonic transducer means,
characterised by the steps of:
analysing the signals in a frequency analysis means included in the confirming means, and
comparing inherent frequency components generated by the calculus responding to the emission of the low powered focused shockwave with a predetermined threshold value in a comparing means and implementing the step of prohibiting the emission when the inherent frequency component does not exceed the threshold value.

The second ultrasonic shockwaves are preferably emitted in every one or several emissions of the first ultrasonic shockwaves.

An embodiment of an apparatus for disintegrating calculus in the living body, and a method of operating the apparatus, will now be described with reference to the accompanying drawings; in which,
Figure 1 is a general diagram of an apparatus according to a preferred embodiment of the invention; and
Figure 2 is a detailed block diagram of signal circuits 21 being used in Figure 1.

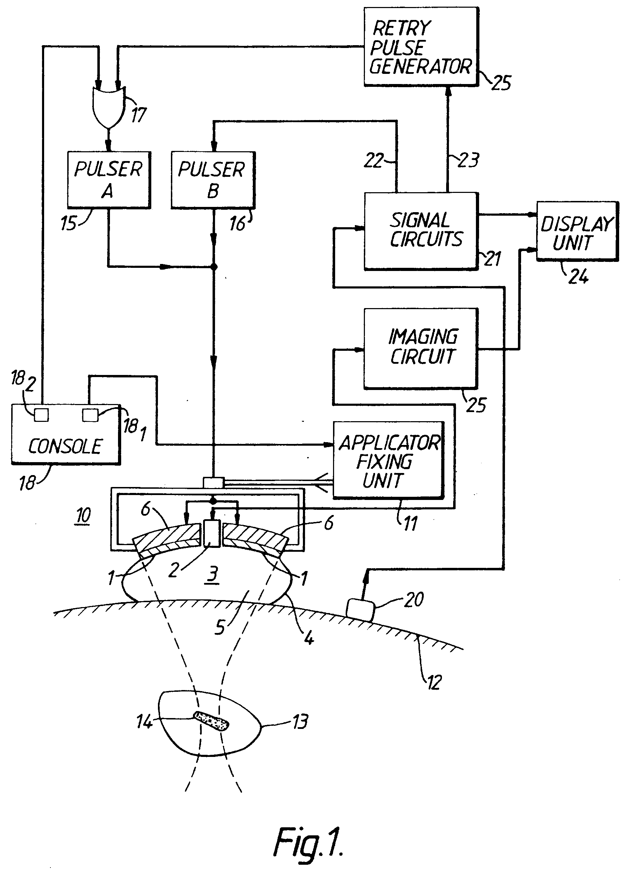

Figure 1 shows a preferred embodiment according to the invention, which is used for disintegrating kidney stones in a patient's kidney.

The apparatus has an applicator 10 and an applicator fixing unit 11. Applicator 10 comprises a main transducer 1, an imaging transducer 2, an acoustic coupler 3 and a housing 4. Applicator 10 is put on the patient's back 12 and is fixed by the applicator fixing unit 11 so as to coincide the geometrical focal point of the main transducer 1 to a kidney stone 14 in the patient's kidney 13.

Applicator fixing unit 11 drives the imaging transducer 2 which emits an ultrasonic wave and receives its echo signals so as to derive a cross-sectional image of the patient. The echo signals are processed by imaging circuits 25, which are the same as those usually used in a conventional ultrasonic diagnostic apparatus. The cross-sectional image is displayed at a display unit 24. The relative positions of the main transducer 1 and the imaging transducer 2 are preferably selected, to localise the geometrical focal point of the main transducer exactly in the cross-sectional plane of the imaging transducer 2. So the operator can recognise whether the kidney stone is coincident with the focal point of the main transducer 1 by watching the cross sectional image obtained by the imaging transducer 2.

The main transducer 1 may be a concave piezoelectric transducer with a curvature of 10 cm in diameter and a 500 kHz resonant frequency, having a backing member 6 adhered to the back surface of the main transducer 1. Main transducer 1 also may be comprised of a plurality of relatively small piezoelectric elements arranged on a concave surface.

Acoustic coupler 3 comprises a bag 4 made of a flexible membrane having substantially the same acoustic impedance as water, and filled with water 5.The acoustic coupler 3 enables an acoustic coupling between the main transducer 1 and the patient's surface, so as to efficiently transmit and receive ultrasonic shockwaves. It is also suitable to provide an acoustic matching layer coated on the surface of piezoelectric transducer having the thickness of λ/4, wherein λ is the wave length of the ultrasonic shockwave, in order to improve efficiency and obtain a short ultrasonic wave.

Pulser-A 15 and pulser-B 16 drive main transducer 1. Pulser-A 15 supplies a drive pulse having relatively low voltage and main transducer 1 emits a weak ultrasonic shockwave for confirming the position of a kidney stone 14. Pulser-B 16 supplies a drive pulse having a high voltage and main transducer 1 emits a high power ultrasonic shockwave for disintegrating the kidney stone 14. Preferably the energy of the weak ultrasonic shockwave is less than 50% of the high power ultrasonic shockwave.

Microphone 20 is provided on the patient's back 12, to collect internal sounds from the patient and convert the sounds into an electric signal. This electric signal is supplied to signal circuits 21. Signal circuits 21 determine whether the calculus is positioned at the focal point
of the main transducer 1 by analysing the electric signal. When the calculus is just at the focal point of the main transducer 1, signal circuits 21 generate a confirmation signal 22. The confirmation signal 22 is supplied to the pulser-B 16 and the pulser-B 16 drives main transducer 1 so as to emit a high power ultrasonic shockwave. On the other hand, when the calculus is not positioned at the focal point of main transducer 1, the signal circuits 21 generate an unconfirmed signal 23.

Localisation of kidney stone to the focal point of main transducer is performed as follows. At first, the operator generally localises the applicator 10 on the patient's back and watches the cross-sectional image of kidney stone 14 on the display unit 24, being obtained by image transducer 2 and imaging circuit 25.

When the operator cannot recognise the kidney stone on the cross sectional image, he then pushes a positioning switch 18₁ from a console 18 to supply a positioning signal to applicator fixing unit 11. Applicator fixing unit 11 then generates a driving signal so as to move applicator 10 corresponding to the positioning signal. After this adjustment, the operator unit 11 fixes the position of the applicator 10.

After the general localisation, a calculus confirmation process is executed. The operator pushes switch 18₂, which generates a control signal to pulser-A 15 through OR gate 17. Thus, pulser-A 15 supplies a drive pulse having a relatively low voltage to the main transducer 1, so that the main transducer 1 emits the weak ultrasonic shockwave. The weak ultrasonic shockwave penetrates or reflects from various tissues inside the patient's body. When the weak ultrasonic shockwave hits a hard material such as a kidney stone in the patient, audio sounds having an inherent frequency of the material are generated. The frequency is dependent upon the kind or size of stone(s). Such audio sounds are received by the microphone 20 and its electric signal is supplied to the signal circuits 21.

The signal circuits 21 analyse the electric signal derived from microphone 20 and confirm whether the kidney stone 14 is precisely at the focal point of the main transducer 1. When the confirmation signal 22 is output from the signal circuits 21, pulser-B 16 is controlled to generate a drive pulse having a large wave height. The drive pulse is supplied to the main transducer 1 and a high power ultrasonic shockwave is emitted in order to disintegrate the kidney stone 14. However, when the unconfirmed signal 23 is output from the signal circuits 21, pulser-B 16 is not controlled and emission of the high power ultrasonic shockwave is prohibited. Responding to the unconfirmed signal 23, retry pulse generator 25 is activated to generate a retry control pulse which is the same pulse as is generated when the switch 18₂ is pushed. This retry control pulse is supplied to pulser-A 15 through OR gate 17. Thus, the weak ultrasonic shockwave is again emitted from transducer 1, and the calculus confirmation process is repeated.

The calculus confirmation process is executed just before the emission of each high power ultrasonic shockwave according to the preferred embodiment, it would also be possible to execute the above calculus confirmation process at every one of several emissions of the high power ultrasonic shockwaves. This confirmation process is provided because the position of calculus may shift slightly because of the patient's respiration or for other reasons, even when its position was previously adjusted to the focal point of the main transducer in the localisation step, for example, by using the cross-sectional image.

Further, according to the preferred embodiment, the emission of the high power ultrasonic shockwave for disintegrating a kidney stone is prohibited when the kidney stone is not at the focal point of the main transducer according to the calculus confirmation process, so that any harmful emission of the high power ultrasonic shockwave to the neighbouring normal tissue is prevented.

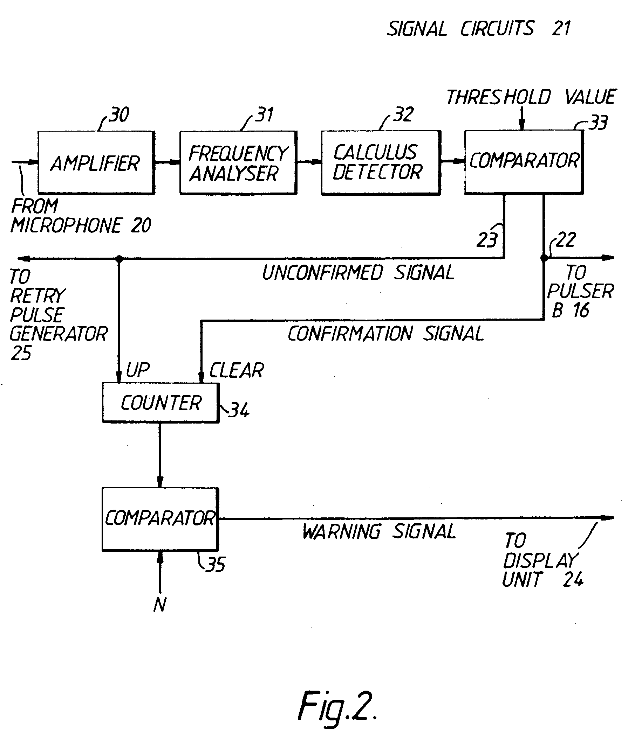

Figure 2 shows a block diagram of signal circuits 21 in Figure 1. Signal circuits 21 comprise an amplifier 30 which amplifies the electric signal derived from the microphone 20, a frequency analyser 31 which extracts the frequency components corresponding to the inherent frequency of the kidney stone, a calculus detector 32 and a comparator 33. The frequency analyser 31 may be a band pass filter for passing only the inherent frequency components and the calculus detector 32 may be a peak value detector or an integrator of the output of the frequency analyser 31. The comparator 33 compares the output of the calculus detector 32 with a threshold value being prepared previously, generating the confirmation signal 22 when the output of the calculus detector 32 is greater than the threshold value and also generating the unconfirmed signal 23 when the output of the calculus detector 32 is smaller than the threshold value.

According to the preferred embodiment, a counter 34 is further provided which counts the number of unconfirmed signals 23. The unconfirmed signal 23 is supplied to the UP terminal of counter 34 to count up their number and confirmed signal 22 is supplied to the CLEAR terminal of the counter 34 so as to clear the content of the counter 34. The content of the counter 34 is compared with a predetermined number N, for example N=3, by comparator 35. If the unconfirmed signals 23 are generated N times consecutively, the comparator 35 generates a warning signal, indicating an unrecovered or permanent position shift of the calculus or it has occurred that the position of the calculus and the focal point of main transducer are no longer coincident . This warning signal is displayed at the display unit 24, so that the operator can again perform the localisation step using cross-sectional image.

The invention is not restricted to the above preferred embodiment. The apparatus according to the embodiment is utilised for disintegrating not only kidney stones but also other calculus. If the microphone has such frequency characteristics that it only senses the inherent frequency components of a calculus, a frequency analyser is not necessary. The microphone may be arranged inside of the applicator. The warning signal may be fed back to the applicator fixing unit 11 so as to automatically move the position of applicator where the level of audio sounds from the calculus is beyond the threshold level.

The high power ultrasonic shockwave and the weak ultrasonic shockwave may be generated by different piezoelectric transducers.

## Claims

1. An apparatus for disintegrating calculus (14) in a living body using ultrasonic shockwaves comprising:
ultrasonic transducer means (1) for emitting first ultrasonic shockwaves having a first energy level for location of the calculus, and for emitting second ultrasonic shockwaves having an energy level, greater than the first energy level, to disintegrate it;
sound receiver means (20) for receiving sound waves from the living body and transforming the sound waves into electrical signals, said sound waves being generated by the calculus in response to said first ultrasonic shockwave; and
confirmation means, coupled to said sound receiver means for confirming the position of the calculus at the focal point of the ultrasonic transducer means by using said electrical signal derived from said sound receiver means (20);
control means, coupled between said confirmation means and said ultrasonic transducer means for prohibiting the emission of the second ultrasonic shockwave when said confirmation means has determined that the position of the calculus is not at the focal point of the ultrasonic transducer means characterised in that said confirmation means includes frequency analysis means for analysing the frequency components corresponding to the inherent frequency of the audio sounds generated by the calculus responding to the emission of the first ultrasonic shockwaves and comparing means (33) for comparing said frequency components with a predetermined threshold value.

2. An apparatus according to claim 1, wherein said comparing means (33) generates a confirmation signal and an unconfirmed signal in accordance with the compared result, and the control means allows the emission of the second ultrasonic shockwave in response to the confirmation signal and prohibits the emission of the second ultrasonic shockwave in response to the unconfirmed signal.

3. An apparatus according to claim 2, wherein said confirmation means includes counter means (34) for counting the number of unconfirmed signals, and warning means (35) for generating a warning signal when the number of unconfirmed signals exceeds a predetermine number.

4. An apparatus according to claim 1, wherein an imaging means (25) is provided for imaging the cross-sectional image of the living body and a display means (24) is provided for displaying the cross sectional image obtained by said imaging means (25).

5. An apparatus according to claim 1 wherein the sound receiver means (20) is a microphone.

6. An ultrasonic lithotrity apparatus according to claim 1, wherein the confirmation means allows the emission of a plurality of second ultrasonic shockwaves to the calculus.

7. A method of operating an apparatus for disintegrating calculus comprising the steps of:
projecting a harmlessly low powered focused shockwave from an ultrasonic transducer means (2),
receiving sound waves with a receiver (20) directed towards the target on which the ultrasonic transducer means (2) is focused and converting the received sound waves into electrical signals,
transmitting the signals to a confirming means,
prohibiting the emission of a second, high energy, calculus disintegrating shockwave from the ultrasonic transducer means,
characterised by the steps of:
analysing the signals in a frequency analysis means (31) included in the confirming means, and
comparing inherent frequency components generated by the calculus responding to the emission of the low powered focused shockwave with a predetermined threshold value in a comparing means (33) and implementing the step of prohibiting the emission when the inherent frequency component does not exceed the threshold value.

## Patentansprüche

1. Vorrichtung zum Zerkleinern von Konkrementen (14) in einem lebenden Körper unter Verwendung von Ultraschall-Druckwellen, mit:
einem Ultraschallwandlermittel (1) zum Aussenden von ersten Ultraschall-Druckwellen mit einem ersten Energiewert, um das Konkrement zu lokalisieren, und zum Aussenden von zweiten Ultraschall-Druckwellen mit einem Energiewert, der größer ist als der erste Energiewert, um es zu zerkleinern;
einem Schallempfängermittel (20) zum Empfangen von Schallwellen aus dem lebenden Körper und zum Umwandeln der Schallwellen in elektrische Signale, wobei die Schallwellen durch das Konkrement in Reaktion auf die erste Ultraschall-Druckwelle erzeuge werden; und
einer Bestätigungseinrichtung, die mit dem Schallempfängermittel verbunden ist, um die Position des Konkrements im Brennpunkt des Ultraschallwandlermittels unter Verwendung der aus dem Schallempfängermittel (20) umgewandelten elektrischen Signale zu bestätigen;
einem Steuermittel, das zwischen der Bestätigungseinrichtung und dem Ultraschallwandlermittel geschaltet ist, um die Aussendung der zweiten Ultraschall-Druckwelle zu verhindern, wenn die Bestätigungseinrichtung festgestellt hat, daß die Position des Konkrements nicht im Brennpunkt des Ultraschallwandlermittels liegt, **dadurch gekennzeichnet**, daß die Bestätigungseinrichtung Frequenzanalysemittel, um die Frequenzkomponenten entsprechend der Eigenfrequenz von durch das Konkrement in Reaktion auf die Aussendung der ersten Ultraschall-Druckwellen erzeugten Schallwellen zu analysieren, und Vergleichsmittel (33) enthält, um die Frequenzkomponenten mit einem vorherbestimmten Schwellenwert zu vergleichen.

2. Vorrichtung nach Anspruch 1, wobei die Vergleichsmittel (33) gemaß dem Vergleichsresultat ein Bestätigungssignal und ein Nicht-Bestätigungssignal erzeugen und die Steuermittel die Aussendung der zweiten Ultraschall-Druckwelle auf das Bestätigungssignal hin erlaubt und die Aussendung der zweiten Ultraschall-Druckwelle auf das Nicht-Bestätigungssignal hin verbietet.

3. Vorrichtung nach Anspruch 2, wobei die Bestätigungseinrichtung Zählermittel (34) zum Zählen der Anzahl von Nicht-Bestätigungssignalen und Warnmittel (35) enthalten, um ein Warnsignal zu erzeugen, wenn die Anzahl von Nicht-Bestätigungssignalen eine vorgegebene Zahl übersteigt.

4. Vorrichtung nach Anspruch 1, wobei ein Abbildungsmittel (25) vorgesehen ist, um das Querschnittsbild des lebenden Körpers zu bilden, und ein Anzeigemittel (24) vorgesehen ist, um das von den Abbildungsmitteln (25) erhaltene Querschnittsbild anzuzeigen.

5. Vorrichtung nach Anspruch 1, wobei das Schallempfängermittel (20) ein Mikrophon ist.

6. Ultraschall-Lithtripsie-Vorrichtung nach Anspruch 1, wobei die Bestätigungseinrichtung die Aussendung einer Mehrzahl von zweiten Ultraschall-Druckwellen zu dem Konkrement erlaubt.

7. Verfahren zum Betreiben einer Vorrichtung zum Zerkleinern von Konkrementen, mit den Schritten:
Ausstrahlen einer fokussierten Druckwelle mit unschädlich niedriger Leistung von einem Ultraschallwandlermittel (2),
Empfangen von Schallwellen mit einem Empfänger (20), welcher auf das Ziel gerichtet ist, auf das das Ultraschallwandlermittel (2) fokussiert ist, und Umwandeln der empfangenen Schallwellen in elektrische Signale,
Senden der Signale zu einer Bestätigungseinrichtung,
Verhindern der Aussendung einer zweiten, energiereicheren, konkrementzerkleinernden Druckwelle von dem Ultraschallwandlermittel,
**gekennzeichnet durch** die Schritte:
Analysieren der Signale in einem Frequenzanalysemittel (31), welches in der Bestätigungseinrichtung enthalten ist, und
Vergleichen von Eigenfrequenzkomponenten, die von dem Konkrement auf die Aussendung der energiearmen fokussierten Druckwelle hin erzeugt werden, mit einem vorgegebenen Schwellenwert in Vergleichsmitteln (33) und Ausführen des Schrittes des Verbietens der Aussendung, wenn die Eigenfrequenzkomponente den Schwellenwert nicht überschreitet.

## Revendications

1. Dispositif pour dissoudre le calcul (14) à l'intérieur du corps d'un patient en utilisant les ondes de choc ultrasonores, comprenant:-
un moyen à transducteur ultrasonique (1) qui émet des premières ondes de choc ultrasonores ayant un premier niveau de puissance pour localiser le calcul, et qui émet des secondes ondes de choc ultrasonores d'une puissance dont le niveau est supérieur à celui du niveau des premières ondes de choc ultrasonores, pour dissoudre ce calcul;
un moyen de réception des sons (20) qui reçoit les ondes sonores envoyées par le corps du patient et qui transforme ces ondes sonores en signaux électriques, ces ondes sonores étant générées par le calcul en réponse à la première onde de choc ultrasonore; et
un moyen de confirmation accouplé à ce moyen de réception des sons (20) pour confirmer que la position du calcul coïncide avec le point de focalisation du moyen à transducteur ultrasonique en utilisant le signal électrique envoyé par ce moyen de réception des sons (20);
un moyen de commande, placé entre ce moyen de confirmation et le moyen à transducteur ultrasonique pour empêcher l'émission d'une seconde onde de choc ultrasonore lorsque ce moyen de confirmation a déterminé que la position du calcul ne coïncide pas avec le point de focalisation du moyen à transducteur ultrasonique, caractérisé par le fait que le moyen de confirmation comporte un moyen d'analyse de fréquence pour analyser les composantes de fréquence correspondant à la fréquence propre à l'onde sonore générée par le calcul en réponse à l'émission des premières ondes de choc ultrasonores et un moyen de comparaison (33) pour comparer les composantes de cette fréquence avec une valeur de seuil prédéterminée.

2. Dispositif selon la revendication 1, dans lequel le moyen de comparaison (33) génère un signal de confirmation et un signal de non confirmation qui est fonction du résultat de la comparaison ci-dessus mentionnée, et le moyen de commande permet à l'émission de la seconde de choc ultrasonore en réponse au signal de confirmation et empêche l'émission de la seconde onde de choc ultrasonore en réponse au signal de non confirmation.

3. Dispositif selon la revendication 2, dans lequel le moyen de confirmation comprend un compteur (34) qui compte le nombre de signaux non confirmés, et un moyen de signalisation (35) qui génère un signal d'alarme lorsque le nombre de signaux non confirmés dépasse un nombre prédéterminé.

4. Dispositif selon la revendication 1, qui dans lequel on a prévu un moyen d'imagerie (25) pour générer une image de la section transversale du corps du patient, et dans lequel on a prévu un moyen d'affichage (24) pour afficher l'image de la coupe transversale ainsi obtenue à l'aide de ce moyen d'imagerie (25).

5. Dispositif selon la revendication 1, dans lequel le moyen de réception des sons (20) est un microphone.

6. Dispositif de lithotritie ultrasonique selon la revendication 1, dans lequel le moyen de confirmation permet d'émettre une pluralité d'ondes de choc ultrasonores vers le calcul.

7. Méthode de fonctionnement d'un dispositif pour dissoudre les calculs comprenant les phases suivantes:-
projection d'une onde de choc ultrasonore de faible puissance sans danger pour le corps humain par un moyen à transducteur ultrasonore (2),
réception d'ondes sonores par un récepteur (20) dirigé vers la cible sur laquelle le moyen à transducteur (2) est pointé et conversion des ondes sonores reçues en signaux électriques,
transmission des signaux à un moyen de confirmation,
empêcher l'émission par le moyen à transducteur ultrasonique d'une seconde onde de choc ultrasonore de grande puissance servant à dissoudre le calcul,
caractérisé par les phases suivantes:-
l'analyse des signaux par un moyen d'analyse de fréquence (31) inclus dans le moyen de confirmation, et
la comparaison entre les composantes de la fréquences propre au calcul généré par le calcul en réponse à l'émission de l'onde de choc ultrasonore de faible puissance et une valeur de seuil prédéterminée dans le moyen de comparaison (33) et mise en oeuvre de la phase qui empêche l'émission lorsque la composante de la fréquence propre au calcul est inférieure ou égale à la valeur de seuil.
